# EUROPÄISCHE PATENTSCHRIFT

(11) **EP 2 915 548 B1**
(45) Veröffentlichungstag und Bekanntmachung des Hinweises auf die Patenterteilung: **01.11.2017**
(21) Anmeldenummer: 15157114.8
(22) Anmeldetag: 02.03.2015
(51) Int. Cl.: A61L 15/60, C08F 20/08, A61L 15/00, C08F 20/06, C08L 33/02, C08F 220/06, C08K 3/30

(54) **SUPERABSORBIERENDE POLYMERE MIT VERBESSERTER GERUCHSKONTROLLEIGENSCHAFT SOWIE VERFAHREN ZU DEREN HERSTELLUNG**
SUPERABSORBENT POLYMERS HAVING IMPROVED ODOUR CONTROL PROPERTIES AND METHOD FOR THE PRODUCTION OF SAME
POLYMÈRES SUPER ABSORBANTS AVEC PROPRIÉTÉ DE CONTRÔLE DES ODEURS AMÉLIORÉE ET LEUR PROCÉDÉ DE FABRICATION

(30) Priorität: 05.03.2014 US 201461948114 P; 05.03.2014 EP 14157784
(43) Veröffentlichungstag der Anmeldung: 09.09.2015
(73) Patentinhaber: Evonik Degussa GmbH, 45128 Essen (DE)
(72) Erfinder: Loick, Christoph, 47918 Tönisvorst (DE); Smith, Scott, 40489 Düsseldorf (DE)

(56) Entgegenhaltungen:
- WO-A2-2010/096595
- DE-A1- 10 218 147

## Beschreibung

Die vorliegende Erfindung betrifft ein Verfahren zur Herstellung superabsorbierende Polymere mit verbesserter Geruchskontrolleigenschaft.

DE 40 20 780 C1 offenbart die Nachbehandlung superabsorbierender Polymere durch Nachvernetzung der Oberflächen der Polymerteilchen. Durch die Nachvernetzung der Oberfläche der wasserabsorbierenden Polymerteilchen wird insbesondere das Absorptionsvermögen der Polymerteilchen unter der Einwirkung von Drücken erhöht.

DE 199 09 653 A1 und DE 199 09 838 A1 beschreiben pulverförmige, an der Oberfläche nachvernetzte, Wasser, wässrige oder seröse Flüssigkeiten oder Blut absorbierende Polymerisate, welche auf säuregruppentragenden Monomeren basieren und welche mit einem Oberflächennachvernetzungsmittel und einem Kation in wässriger Lösung beschichtet und nachvernetzt worden sind. Die in diesem Stand der Technik offenbarten Polymerisate weisen gegenüber herkömmlichen Polymerisaten vorteilhafte Absorptionseigenschaften, insbesondere eine hohe Permeabilität auf.

Hygieneartikel wie Windeln oder Inkontinenzprodukte, Damenbinden und andere Artikel, die Superabsorbierende Polymere enthalten, werden eingesetzt, um Körperflüssigkeiten wie Urin aufzunehmen. Urin ist jedoch eine Quelle für Schlechtgerüche, einerseits durch bakterielle und enzymatische Zersetzung des Urins, bei welcher Ammoniak entsteht, andererseits auch durch den Geruch von organischen Verbindungen im Urin selbst. Ammoniakgeruch entsteht nach einer gewissen Zeit, wobei der Zeitraum der benötigt wird, um Urin in Ammoniak umzusetzen von vielen Faktoren wie beispielsweise den Bakterien auf der Hautoberfläche, dem pH-Wert, der Temperatur und anderem abhängig ist. Gerüche, die durch organische Verbindungen hervorgerufen werden, beispielsweise Nebenprodukte des Stoffwechsels oder anderen metabolischen Prozessen, sind demgegenüber sofort wahrnehmbar, nachdem der Urin aus dem Körper ausgeschieden wurde. Es besteht ein großer Bedarf beide Arten der Gerüche zu kontrollieren, zu reduzieren und bestenfalls vollständig zu eliminieren.

Beim längeren Tragen von absorbierende Polymere beinhaltenden Hygieneartikeln, insbesondere wenn diese bereits zu einem Teil Körperflüssigkeiten wie Urin aufgenommen haben, kann es bedingt durch die organischen Bestandteile des Urins und die Körperwärme der Trägerperson alsbald zu einer unangenehmen Geruchsbelastung kommen. Um dieser zu begegnen, wurden zahlreiche Versuche unternommen, durch entsprechende Beigaben in den vom Superabsorber verschiedenen Bestandteilen des Hygieneartikels eine Bindung der geruchsbildenden Stoffe zu erzielen oder den Geruch durch Parfums oder dergleichen zu übertönen. Das Einbringen von derartigen Substanzen in der Form von Superabsorbern verschiedenen Bestandteilen wirkt sich oftmals negativ auf die Performance dieser Hygieneartikel während des Tragens aus. So werden durch die Körperflüssigkeiten oftmals die anfänglich vom Superabsorberbereich räumlich getrennt vorliegenden geruchshemmenden oder geruchsvermindernden Substanzen beispielsweise durch Hineinschwemmen in Superabsorber beinhaltende Bereich eines Hygieneartikels eingetragen, wo sie dann eine negative Auswirkung auf die Performance des Superabsorbers und damit des Hygieneartikels insgesamt zeigen. Ferner ist an diesem Konzept nachteilig, dass der Großteil der in den Hygieneartikel abgegebenen Körperflüssigkeit sich ohnehin in dem Superabsorber befindet und daher die außerhalb des Superabsorbers befindlichen geruchshemmenden oder geruchsvermindernden Substanzen ihre Wirkung schlechter entfalten können.

DE 198 25 486 und DE 199 39 662 A1 offenbaren die Kombination von Superabsorbern mit Cyclodextrin zur Geruchsverminderung. Diesem durchaus viel versprechenden Ansatz ist jedoch zu entnehmen, dass das Cyclodextrin nur unter bestimmten Bedingungen, nämlich wenn sichergestellt ist, dass das Cyclodextrin nicht von dem Superabsorber wieder separiert, seine geruchshemmende Wirkung in dem Superabsorber zeigt. Hierbei ist es bevorzugt, dass das Cyclodextrin zumindest in die Oberfläche des Superabsorberartikels eingearbeitet ist, indem Cyclodextrin und/oder Cyclodextrinderivate kovalent und/oder ionisch gebunden und/oder darin eingeschlossen sind.

Aus DE 103 34 271 sind weiterhin Superabsorberagglomerate bekannt, die eine Vielzahl von Geruchsbindern homogen in dem Agglomerat aufweisen können. Diese eine hervorragende Lösung für die Verwendung von Superabsorberfeinteilchen offenbarende Schrift stellt jedoch keine für die Anwendung in Hygieneartikeln besonders gut geeignete Superabsorber mit Geruchsbindungseigenschaften zur Verfügung. So sind neben einem effizienten und wirkungsvollen Einsatz der Geruchsbinder auch die durch diesen Geruchsbinder beeinflussten Superabsorbereigenschaften noch verbesserungswürdig.

DE-A-10 2005 055 497 lehrt, superabsorbierende Polymere dadurch das in Kontakt bringen mit Metallsalzen der Ricinolsäure und/oder mit Aminosäuren mit verbesserten geruchsbindenden Eigenschaften zu versehen.

Ebenfalls mit geruchsinhibierten Superabsorbern befasst sich WO2011023560A2. Nach deren Lehre werden vorzugsweise Zink-, Magnesium-, Calcium,- oder Lithiumperoxid den Superabsorbern eingesetzt. Die besten Ergebnisse werden dabei mit Zinkperoxid erzielt.

Weitere Ansätze, wie beispielsweise die Reduzierung des pH-Wertes, Enzyminhibitoren, Chelatbildner und ähnliches, zeigen sich lediglich darin vorteilhaft, dass sie die Freisetzung von Ammoniak verzögern nicht jedoch verhindern konnten, während sie keinen Einfluss auf die Gerüche aus organischen Verbindungen haben oder lediglich eine kleine Substanzklasse von organischen Verbindungen binden können. Eine besondere Herausforderung darin, Gerüche, insbesondere Schlechtgerüche zu inhibieren, stellt die Vielzahl an Verbindungen mit unterschiedlichen chemischen Eigenschaften, wie beispielsweise Ketone, Aldehyde, Amine, Mercaptane, Sufide, zyklische Verbindungen, ungesättigte Verbindungen, Aromaten und so weiter, dar. Werden nur einige der wahrnehmbaren Gerüche inhibiert, so wirkt sich dies wenig bis gar nicht auf die Wahrnehmung des Gesamtgeruches also auf den Eindruck des Schlechtgeruches aus, der von Urin verursacht wird.

Im Allgemeinen lag der vorliegenden Erfindung die Aufgabe zugrunde, die sich aus dem Stand der Technik ergebenden Nachteile abzumildern oder gar zu überwinden.

Eine erfindungsgemäße Aufgabe bestand darin, ein wasserabsorbierendes Polymer bereitzustellen, welches zum einen über gute Geruchsbindungseigenschaften und Geruchsreduzierungsigenschaften verfügt. Zudem betseht bedarf an einem wasserabsorbierenden Polymer, mit vielfältigen Geuchskontrolleigenschaften, mit anderen Worten welches Gerüche sowohl von Ammoniak als auch von anderen organischen verbindugen reduzieren oder inhibieren kann. Die Geuchskontrolleigenschaften sollen sich dabei auf eine Vielzahl organischer Geruchsstoffe auswirken, aus mehr als nur ein oder zwei Substanzklassen. Dabei soll zum anderen gewährleistet sein, dass die Performance des Hygieneartikels, der dieses geruchsbindende oder geruchsreduzierende wasserabsorbierende Polymer beinhaltet, im Wesentlichen gleich gut oder gar besser ist als die Performance des Hygieneartikels mit einem Superabsorber, der nicht wie das geruchsbindende wasserabsorbierende Polymer den Geruchsbinder beinhaltet. Insbesondere sollte die Performance-Eigenschaften des wasserabsorbierenden Polymers durch die Verwendung geruchsbindender Additive, welche in möglichst geringen Mengen eingesetzt werden sollen, möglichst gar nicht oder allenfalls geringfügig beeinflusst werden. Vorteilhafterweise sollten die Performance-Eigenschaften des wasserabsorbierenden Polymeren durch den Zusatz des geruchsbindenden oder geruchsreduzierenden Additivs sogar verbessert werden.

Weiterhin sollte das wasserabsorbierende Polymer bei einem Kontakt mit wässrigen Körperflüssigkeiten, insbesondere mit Urin oder eisenhaltigen Flüssigkeiten, wie beispielsweise Blut oder Menstruationsflüssigkeit, möglichst nicht zu starken Verfärbungen neigen.

Ferner bestand eine erfindungsgemäße Aufgabe darin, ein Verfahren aufzuzeigen, mit dem ein solches wasserabsorbierende Polymer erhalten werden kann. Zudem bestand eine erfindungsgemäße Aufgabe darin, einen Hygieneartikel bereitzustellen, der neben guten geruchsbindenden oder geruchsreduzierenden Eigenschaften in Bezug auf eine Vielzahl von aus dem Urin resultierenden Schlechtgerüchen auch eine gute Performance zeigt. Außerdem lag der vorliegenden Erfindung als Aufgabe zugrunde, wasserabsorbierende Polymere bereitzustellen, die sich allgemein in Verbunde einarbeiten lassen oder auch als Verbund oder als solche Verwendung in chemischen Produkten oder deren Bestandteilen finden können.

Diese Aufgaben werden gelöst durch den Gegenstand des Anspruch 1. Vorteilhafte Ausgestaltungen und Weiterbildungen, die einzeln oder in Kombination auftreten können, sind Gegenstand der abhängigen Ansprüche.

Als Peroxoverbindungen werden solche aus der Gruppe von organischen oder anorganischen Peroxoverbindungen verstanden.

"Anorganische Peroxoverbindungen" sind beispielsweise solche aus der Gruppe von Alkali- oder Erdalkali Peroxomonosulfat, oder Peroxodisulfat. Beispielhafte anorganische Peroxoverbindungen sind ausgewählt aus der Gruppe aus von Alkali- oder Erdalkali Peroxomonosulfat, Peroxodisulfat, Sulfomonopersäure, Peroxomonosulfat-triple Salz (Caro'sche Säure) oder Mischungen daraus. Erfindungsgemäß wird Peroxomonosulfat-triple Salz eingesetzt.

Unter dem Begriff "organische Peroxoverbindungen" werden solche aus der Gruppe von Peroxocarbonsäuren verstanden. Beispielhafte organische Peroxoverbindungen sind ausgewählt aus der Gruppe aus Carbamidperoxo, Peroxocarbonsäuren oder Mischungen daraus.

Wasserabsorbierende Polymere, die mit der erfindungsgemäßen Peroxoverbindung behandelt oder in Kontakt gebracht wurden, weisen exzellente geruchsbindende und geruchsreduzierende Eigenschaften auf, sowohl in Bezug auf Ammoniak-Gerüche als auch in Bezug auf Gerüche aus organischen Verbindungen. Die Anwendungseigenschaften der Hygieneartikel, die mit der erfindungsgemäßen Peroxoverbindung behandelte wasserabsorbierende Polymerpartikel beinhalten, sind fast ausnahmslos genauso gut oder sogar besser als die solcher Hygieneartikel, die wasserabsorbierende Polymerpartikel ohne Peroxoverbindungen beinhalten. Überaschenderweise, weisen die wasserabsorbierenden Polymerpartikel auch keine oder falls überhaupt lediglich schwache Verfärbungen auf. Die besten Ergebnisse konnten mit anorganischen Peroxoverbindungen, erzielt werden. Insbesondere der Einsatz anorganischer Peroxoverbindungen ausgewählt aus der Gruppe aus von Alkali- oder Erdalkali Peroxomonosulfat, Peroxodisulfat, Sulfomonopersäure, Peroxomonosulfat-triple Salz (Caro'sche Säure) oder Mischungen daraus, besonders Peroxomonosulfat-triple Salz, in den vorab angegeben bevorzugten Gew.-%, führt zu wasserabsorbierenden Polymerpartikeln mit hervorragenden Geruchskontrolleigenschaften.

Die Salze der anorganischen Peroxoverbindungen umfassen Salze, die aus der Gruppe der Alkali-, Erdalkali und der Borgruppe stammen. Hierbei sind bevorzugt die Metalle aus der Gruppe von Natrium, Kalium, Cäsium, Rubidium, Magnesium, Calcium, Strontium, Barium, Aluminium, Gallium, Indium. Besonders zu erwähnen sind solche aus der Gruppe von Natrium, Kalium, Calcium, Magnesium gegebenenfalls Mischungen dieser. Besonders zu erwähnen sind Metalle aus der Gruppe von Natrium, Kalium Calcium und Magnesium.

Dabei sind erfindungsgemäß bevorzugte wasserabsorbierende Polymergebilde insbesondere Fasern, Schäume oder Teilchen, wobei Fasern und Teilchen besonders bevorzugt und Teilchen am meisten bevorzugt sind.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Erfindungsgemäß bevorzugte Polymerteilchen sind so dimensioniert, dass sie eine mittlere Teilchengröße gemäß ERT 420.2-02 im Bereich von 10 bis 3000 µm, vorzugsweise 20 bis 2000 µm und besonders bevorzugt 150 bis 850 µm oder 150 bis 600 µm aufweisen. Dabei ist es insbesondere bevorzugt, dass der Anteil der Polymerteilchen mit einer Partikelgröße in einem Bereich von 300 bis 600 µm mindestens 30 Gew.-%, besonders bevorzugt mindestens 40 Gew.-%, darüber hinaus bevorzugt mindestens 50 Gew.-% und am meisten bevorzugt mindestens 75 Gew.-%, bezogen auf das Gesamtgewicht der wasserabsorbierenden Polymerteilchen, beträgt. Gemäß einer anderen Ausführungsform der erfindungsgemäßen wasserabsorbierenden Polymergebilde beträgt der Anteil der Polymerteilchen mit einer Partikelgröße in einem Bereich von 150 bis 850 µm mindestens 50 Gew.-%, besonders bevorzugt mindestens 75 Gew.-%, darüber hinaus bevorzugt mindestens 90 Gew.-% und am meisten bevorzugt mindestens 95 Gew.-%, bezogen auf das Gesamtgewicht der wasserabsorbierenden Polymerteilchen.

Die monoethylenisch ungesättigten, säuregruppenhaltigen Monomere (α1) können teilweise oder vollständig, bevorzugt teilweise neutralisiert sein. Vorzugsweise sind die monoethylenisch ungesättigten, säuregruppenhaltigen Monomere zu mindestens 10 Mol-%, besonders bevorzugt zu mindestens 25 bis 50 Mol-% und darüber hinaus bevorzugt zu 50-90 Mol-% neutralisiert. Die Neutralisation der Monomere (α1) kann vor, aber auch nach der Polymerisation erfolgen. Hierbei erfolgt die Teilneutralisierung zu mindestens 10 Mol-%, besonders bevorzugt zu mindestens 25 bis 50 Mol-% und darüber hinaus bevorzugt zu 50-90 Mol-%. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als *"Mixed-Bed Ion-Exchange Absorbent Polymers"* (MBIEA-Polymere) bezeichnet und sind unter anderem in der WO 99/34843 offenbart. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin, sowie deren sekundäre oder tertiäre Aminderivate.

Bevorzugte monoethylenisch ungesättigte, säuregruppenhaltige Monomere (α1) sind Acrylsäure, Methacrylsäure, Ethacrylsäure, α-Chloracrylsäure, α-Cyanoacrylsäure, β-Methylacrylsäure (Crotonsäure), α-Phenylacrylsäure, β-Acryloxypropionsäure, Sorbinsäure, α-Chlorsorbinsäure, 2'-Methylisocrotonsäure, Zimtsäure, p-Chlorzimtsäure, β-Stearylsäure, Itaconsäure, Citraconsäure, Mesaconsäure, Glutaconsäure, Aconitsäure, Maleinsäure, Fumarsäure, Tricarboxyethylen und Maleinsäureanhydrid, wobei Acrylsäure sowie Methacrylsäure besonders und Acrylsäure darüber hinaus bevorzugt sind.

Neben diesen carboxylatgruppenhaltigen Monomeren sind als monoethylenisch ungesättigte, säuregruppenhaltige Monomere (α1) des Weiteren ethylenisch ungesättigte Sulfonsäuremonomere oder ethylenisch ungesättigte Phosphonsäuremonomere bevorzugt.

Als ethylenisch ungesättigte Sulfonsäuremonomere sind Allylsulfonsäure oder aliphatische oder aromatische Vinylsulfonsäuren oder acrylische oder methacrylische Sulfonsäuren bevorzugt. Als aliphatische oder aromatische Vinylsulfonsäuren sind Vinylsulfonsäure, 4-Vinylbenzylsulfonsäure, Vinyltoluolsulfonsäure und Stryrolsulfonsäure bevorzugt. Als Acryl- bzw. Methacrylsulfonsäuren sind Sulfoethyl(meth)acrylat, Sulfopropyl(meth)acrylat, 2-Hydroxy-3-methacryloxypropylsulfonsäure sowie (Meth)Acrylamidoalkylsulfonsäuren wie 2-Acrylamido-2-methylpropansulfonsäure bevorzugt.

Als ethylenisch ungesättigte Phosphonsäuremonomere sind Vinylphosphonsäure, Allylphosphonsäure, Vinylbenzylphosponsäure, (Meth)acrylamidoalkylphosphonsäuren, Acrylamidoalkyldiphosphonsäuren, phosponomethylierte Vinylamine und (Meth)acryl-phosphonsäurederivate bevorzugt.

Als ethylenisch ungesättigte, einen protonierten Stickstoff enthaltende Monomere (α1) sind vorzugsweise Dialkylaminoalkyl(meth)acrylate in protonierter Form, beispielsweise Dimethylaminoethyl(meth)acrylat-Hydrochlorid oder Dimethylaminoethyl(meth)acrylat-Hydro-sulfat, sowie Dialkylaminoalkyl-(meth)acrylamide in protonierter Form, beispielsweise Dimethylaminoethyl(meth)acrylamid-Hydrochlorid, Diemethylaminopropyl(meth)acrylamid-Hydrochlorid, Diemethylaminopropyl(meth)acrylamid-Hydrosulfat oder Dimethylaminoethyl-(meth)acrylamid-Hydrosulfat bevorzugt.

Als ethylenisch ungesättigte, einen quarternierten Stickstoff enthaltende Monomere (α1) sind Dialkylammoniumalkyl(meth)acrylate in quarternisierter Form, beispielsweise Trimethylam-moniumethyl(meth)acrylat-Methosulfat oder Dimethylethylammoniumethyl(meth)acrylat-Etho-sulfat sowie (Meth)acrylamido-alkyldialkylamine in quarternisierter Form, beispielsweise (Meth)acrylamidopro- pyltrimethylammoniumchlorid, Trimethylammoniumethyl(meth)acrylat-Chlorid oder (Meth)acrylamidopropyltrimethylammoniumsulfat bevorzugt.

Als monoethylenisch ungesättigte, mit (α1) copolymerisierbare Monomere (α2) sind Acrylamide und Methacrylamide bevorzugt.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino(meth)- acrylamid, Dimethyl(meth)acrylamid oder Diethyl(meth)acrylamid Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäurester und Methacrylsäurester, wie Methyl(meth)acrylat, Ethyl(meth)acrylat, Propyl(meth)acrylat oder Butyl(meth)acrylat, sowie Vinylacetat, Styrol und Isobutylen bevorzugt.

Erfindungsgemäß bevorzugte Vernetzer (α3) sind Verbindungen, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren (α1) oder (α2) in einer Kondensationsreaktion (=Kondensationsvernetzer), in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV). Dabei wird durch die Verbindungen der Vernetzerklasse I eine Vernetzung der Polymere durch die radikalische Polymerisation der ethylenisch ungesättigten Gruppen des Vernetzermoleküls mit den monoethylenisch ungesättigten Monomeren (α1) oder (α2) erreicht, während bei den Verbindungen der Vernetzerklasse II und den polyvalenten Metallkationen der Vernetzerklasse IV eine Vernetzung der Polymere durch Kondensationsreaktion der funktionellen Gruppen (Vernetzerklasse II) bzw. durch elektrostatische Wechselwirkung des polyvalenten Metallkations (Vernetzerklasse IV) mit den funktionellen Gruppen der Monomere (α1) oder (α2) erreicht wird. Bei den Verbindungen der Vernetzerklasse III erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren (α1) oder (α2).

Bevorzugte Verbindungen der Vernetzerklasse I sind Poly(meth)acrylsäureester, die beispielsweise durch die Umsetzung eines Polyols, wie beispielsweise Ethylenglykol, Propylenglykol, Trimethylolpropan, 1,6-Hexandiol, Glycerin, Pentaerythrit, Polyethylenglykol oder Polypropylenglykol, eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Als Verbindungen der Vernetzerklasse I sind des Weiteren Polyvinylverbindungen, Poly(meth)allyverbindungen, (Meth)acrylsäureester einer Monovinylverbindung oder (Meth)acrylsäureester einer Mono(meth)allyl- verbindung, vorzugsweise der Mono(meth)allylverbindungen eines Polyols oder eines Aminoalkohols, bevorzugt. In diesem Zusammenhang wird auf DE 195 43 366 und DE 195 43 368 verwiesen.

Als Verbindungen der Vernetzerklasse I seien als Beispiel genannt Alkenyldi(meth)acrylate, beispielsweise Ethylenglykoldi(meth)acrylat, 1,3-Propylenglykoldi(meth)acrylat, 1,4-Butylen-glykoldi(meth)acrylat, 1,3-Butylenglykoldi(meth)acrylat, 1,6-Hexandioldi(meth)acrylat, 1,10-Decandioldi(meth)acrylat, 1,12-Dodecandioldi(meth)acrylat, 1,18-Octadecandioldi(meth)acrylat, Cyclopentandioldi(meth)acrylat, Neopentylglykoldi(meth)acrylat, Methylendi(meth)acrylat oder Pentaerythritdi(meth)acrylat, Alkenyldi(meth)acrylamide, beispielsweise N-Methyldi(meth)acrylamid, N,N'-3-Methylbutylidenbis(meth)acrylamid, N,N'-(1,2-Di-hydroxy-ethylen)bis(meth)acrylamid, N,N'-Hexamethylenbis(meth)acryl-acrylamid oder N,N'-Methylenbis(meth)acrylamid, Polyalkoxydi(meth)acrylate, beispielsweise Diethylenglykoldi-(meth)acrylat, Triethylenglykoldi(meth)acrylat, Tetraethylenglykoldi(meth)acrylat, Dipropylenglykoldi(meth)acrylat, Tripropylenglykoldi(meth)acrylat oder Tetrapropylenglykoldi-(meth)acrylat, Bisphenol-A-di(meth)acrylat, ethoxyliertes Bisphenol-A-di(meth)acrylat, Benzylidindi(meth)acrylat, 1,3-Di(meth)acryloyloxy-propanol-2, Hydrochinondi(meth)acry- lat, Di(meth)acrylatester des vorzugsweise mit 1 bis 30 Mol Alkylenoxid pro Hydroxylgruppe oxyalkylierten, vorzugsweise ethoxylierten Trimethylolpropans, Thioethylenglykoldi(meth)-acrylat, Thiopropylenglykoldi(meth)acrylat, Thiopolyethylenglykoldi(meth)acrylat, Thiopoly-propylenglykoldi(meth)acrylat, Divinylether, beispielsweise 1,4-Butandioldivinylether, Divinylester, beispielsweise Divinyladipat, Alkandiene, beispielsweise Butadien oder 1,6-Hexadien, Divinylbenzol, Di(meth)allylverbindungen, beispielsweise Di(meth)allylphthalat oder Di(meth)allylsuccinat, Homo- und Copolymere von Di(meth)allyldimethylammonium-chlorid und Homo- und Copolymere von Diethyl(meth)allyl- aminomethyl(meth)acrylat-ammoniumchlorid, Vinyl-(meth)acryl-Verbindungen, beispielsweise Vinyl(meth)acrylat, (Meth)allyl-(meth)acryl-Verbindungen, beispielsweise (Meth)allyl(meth)acrylat, mit 1 bis 30 Mol Ethylenoxid pro Hydroxylgruppe ethoxyliertes (Meth)allyl(meth)acrylat, Di(meth)allylester von Polycarbonsäuren, beispielsweise Di(meth)allylmaleat, Di(meth)allyfumarat, Di(meth)allylsuccinat oder Di(meth)allylterephthalat, Verbindungen mit 3 oder mehr ethylenisch ungesättigten, radikalisch polymerisierbaren Gruppen wie beispielsweise Glycerintri(meth)acrylat, (Meth)acrylatester des mit vorzugsweise 1 bis 30 Mol Ethylenoxid pro Hydroxylgruppe oxyethylierten Glycerins, Trimethylolpropantri(meth)acrylat, Tri(meth)acrylatester des vorzugsweise mit 1 bis 30 Mol Alkylenoxid pro Hydroxylgruppe oxyalkylierten, vorzugsweise ethoxylierten Trimethylolpropans, Trimethacrylamid, (Meth)allylidendi(meth)acrylat, 3-Allyloxy-1,2-propandioldi(meth)acrylat, Tri- (meth)allylcyan-urat, Tri(meth)allylisocyanurat, Pentaerythrittetra(meth)acrylat, Pentaerythrittri(meth)acrylat, (Meth)acrylsäureester des mit vorzugsweise 1 bis 30 Mol Ethylenoxid pro Hydroxylgruppe oxyethylierten Pentaerythrits, Tris(2-hydroxyethyl)isocyanurattri(meth)acrylat, Trivinyltrimellitat, Tri(meth)allylamin, Di(meth)allylalkylamine, beispielsweise Di(meth)allylmethylamin, Tri-(meth)allylphosphat, Tetra(meth)allylethylendiamin, Poly(meth)allylester, Tetra(meth)allyloxi-ethan oder Tetra(meth)allylammoniumhalide.

Als Verbindung der Vernetzerklasse II sind Verbindungen bevorzugt, die mindestens zwei funktionelle Gruppen aufweisen, die in einer Kondensationsreaktion (=Kondensaionsvernetzer), in einer Additionsreaktion oder in einer Ringöffnungsreaktion mit den funktionellen Gruppen der Monomere (α1) oder (α2), bevorzugt mit Säuregruppen, der Monomeren (a1), reagieren können. Bei diesen funktionellen Gruppen der Verbindungen der Vernetzerklasse II handelt es sich vorzugsweise um Alkohol-, Amin-, Aldehyd-, Glycidyl-, Isocyanat-, Carbonat- oder Epichlorfunktionen.

Als Verbindung der Vernetzerklasse II seien als Beispiele genannt Polyole, beispielsweise Ethylenglykol, Polethylenglykole wie Diethylenglykol, Triethylenglykol und Tetraethylenglykol, Propylenglykol, Polypropylenglykole wie Dipropylenglykol, Tripropylenglykol oder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin, Diethanolamin, Triethanolamin oder Propanolamin, Polyaminverbindungen, beispielsweise Ethylendiamin, Diethylentriaamin, Triethylentetraamin, Tetraethylenpentaamin oder Pentaethylenhexaamin, Polyglycidylether-Verbindungen wie Ethylenglykoliglycidylether, Polyethylenglykoldiglycidylether, Glycerindiglycidylether, Glycerinpolyglycidyether, Pentareritritpolyglycidylether, Propylenglykoldiglycidylether Polypropylenglykoldiglycidylether, Neopentylglykoldiglycidylether, Hexandiolglycidylether, Trimethylolpropanpolyglycidylether, Sorbitolpolyglycidylether, Phtahlsäurediglycidylester, Adipinsäurediglycidylether, 1,4-Phenylen-bis(2-oxazolin), Glycidol, Polyisocyanate, vorzugsweise Diisocyanate wie 2,4-Toluoldiisocyanat und Hexamethylendiisocyanat, Polyaziridin-Verbindungen wie 2,2-Bishydroxymethylbutanol-tris[3-(1-aziridinyl)propionat], 1,6-Hexamethylendiethylenharnstoff und Diphenylmethan-bis-4,4'-N,N'-diethylenharnstoff, Halogenperoxide beispielsweise Epichlor- und Epibromhydrin und α-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, polyquartäre Amine wie Kondensationsprodukte von Dimethylaminen und Epichlorhydrin. Als Verbindungen der Vernetzerklasse II sind des Weiteren Polyoxazoline wie 1,2-Ethylenbisoxazolin, Vernetzer mit Silangruppen wie γ-Glycidoxypropyltrimethoxysilan und γ-Aminopropyltrimethoxysilan, Oxazolidinone wie 2-Oxazolidinon, Bis- und Poly-2-oxazolidinone und Diglykolsilikate bevorzugt.

Als Verbindungen der Klasse III sind hydroxyl- oder aminogruppenhaltige Ester der (Meth)acrylsäure, wie beispielsweise 2-Hydroxyethyl(meth)acrylat und 2-Hydroxypropyl-(meth)acrylat sowie hydroxyl- oder aminogruppenhaltige (Meth)acrylamide oder Mono(meth)allylverbindungen von Diolen bevorzugt.

Die polyvalenten Metallkationen der Vernetzerklasse IV leiten sich vorzugsweise von ein- oder mehrwertigen Kationen ab, die einwertigen insbesondere von Alkalimetallen, wie Kalium, Natrium, Lithium, wobei Lithium bevorzugt wird. Bevorzugte zweiwertige Kationen leiten sich von Zink, Beryllium, Erdalkalimetallen, wie Magnesium, Calcium, Strontium ab, wobei Magnesium bevorzugt wird. Weiter erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z. B. AlCl₃ × 6H₂O, NaAl(SO₄)₂ × 12 H₂O, KAl(SO₄)₂ × 12 H₂O oder Al₂(SO₄)₃×14-18 H₂O eingesetzt. Besonders bevorzugt werden Al₂(SO₄)₃ und seine Hydrate als Vernetzer der Vernetzungsklasse IV verwendet.

Die im erfindungsgemäßen Verfahren eingesetzten Superabsorberteilchen sind vorzugsweise durch Vernetzer der folgenden Vernetzerklassen bzw. durch Vernetzer der folgenden Kombinationen von Vernetzerklassen vernetzt: I, II, III, IV, I II, I III, I IV, I 11 III, I II IV, I III IV, II III IV, II IV oder III IV. Die vorstehenden Kombinationen von Vernetzerklassen stellen jeweils eine bevorzugte Ausführungsform von Vernetzern eines im erfindungsgemäßen Verfahren eingesetzten Superabsorberteilchens dar.

Weitere bevorzugte Ausführungsformen der im erfindungsgemäßen Verfahren eingesetzten Superabsorberteilchen sind Polymere, die durch einen beliebigen der vorstehend genannten Vernetzer der Vernetzerklasse I vernetzt sind. Unter diesen sind wasserlösliche Vernetzer bevorzugt. In diesem Zusammenhang sind N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (α4) können in den Superabsorberteilchen wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsstoffe (α5) sind in den Polymeren, organische oder anorganische Partikel wie beispielsweise Geruchsbinder, insbesondere Zeolithe oder Cyclodextrine, Hautpflegesubstanzen, oberflächenaktive Mittel oder Antioxidantien enthalten.

Zu den bevorzugten organischen Hilfsstoffen gehören Cyclodextrine oder deren Derivate sowie Polysaccharide. Außerdem sind Cellulose und Cellulosederivate wie CMC, Celluloseether bevorzugt. Als Cyclodextrine oder Cyclodextrinderivate sind dabei diejenigen Verbindungen bevorzugt, die in DE-A-198 25 486 auf der Seite 3, Zeile 51 bis Seite 4, Zeile 61 offenbart sind. Besonders bevorzugte Cyclodextrine sind nicht derivatisierte α-, β-, γ- oder δ-Cyclodextrine.

Als anorganische partikuläre Hilfsstoffe können alle Materialen eingesetzt werden, welche üblicherweise zur Modifizierung der Eigenschaften wasserabsorbierender Polymere eingesetzt werden. Zu den bevorzugten anorganischen Hilfsstoffen gehören Sulfate wie Na₂SO₄, Lactate wie etwa Natriumlactat, Silikate, insbesondere Gerüstsilikate wie Zeolithe oder Silikate, die durch Trocknung wässriger Kieselsäurelösungen oder Kieselsolen erhalten wurden, beispielsweise die kommerziell erhältlichen Produkte wie Fällungskieselsäuren und pyrogene Kieselsäuren, beispielsweise Aerosile mit einer Teilchengröße im Bereich von 5 bis 50 nm, vorzugsweise im Bereich von 8 bis 20 nm wie "Aerosil 200" der Evonik Industries AG, Aluminate, Titandioxide, Zinkoxide, Tonmaterialien und weitere dem Fachmann geläufige Mineralien sowie kohlenstoffhaltige anorganische Materialien.

Bevorzugte Silikate sind alle natürlichen oder synthetischen Silikate, die in Hollemann und Wiberg, Lehrbuch der Anorganischen Chemie, Walter de Gruyter-Verlag, 91.-100. Auflage, 1985 auf den Seiten 750 bis 783, als Silikate offenbart sind.

Besonders bevorzugte Silikate sind die Zeolithe. Als Zeolithe können alle dem Fachmann bekannten synthetischen oder natürlichen Zeolithe eingesetzt werden. Bevorzugte natürliche Zeolithe sind Zeolithe aus der Natrolith-Gruppe, der Harmoton-Gruppe, der Mordenit-Gruppe, der Chabasit-Gruppe, der Faujasit-Gruppe (Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabasit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmoton, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS®.

Als Zeolithe können Zeolithe des so genannten "mittleren" Typs eingesetzt werden, bei denen das SiO₂/AlO₂-Verhältnis kleiner als 10 ist, besonders bevorzugt liegt das SiO₂/AlO₂-Verhältnis dieser Zeolithe in einem Bereich von 2 bis 10. Neben diesen "mittleren" Zeolithen können weiterhin Zeolithe des "hohen" Typs eingesetzt werden, zu denen beispielsweise die bekannten "Molekularsieb"-Zeolithe des Typs ZSM sowie β-Zeolith gehören. Diese "hohen" Zeolithe sind vorzugsweise durch ein SiO₂/AlO₂-Verhältnis von mindestens 35, besonders bevorzugt von einem SiO₂/AlO₂-Verhältnis in einem Bereich von 200 bis 500 gekennzeichnet.

Als Aluminate werden vorzugsweise die in der Natur vorkommenden Spinelle, insbesondere gewöhnlicher Spinell, Zinkspinell, Eisenspinell oder Chromspinell eingesetzt.

Bevorzugtes Titandioxid ist das reine Titandioxid in den Kristallformen Rutil, Anatas und Brookit, sowie eisenhaltige Titandioxide wie beispielsweise Ilmenit, calciumhaltige Titandioxide wie Titanit oder Perowskit.

Bevorzugte Tonmaterialien sind diejenigen, die in Hollemann und Wiberg, Lehrbuch der Anorganischen Chemie, Walter de Gruyter-Verlag, 91.-100. Auflage, 1985 auf den Seiten 783 bis 785, als Tonmaterialien offenbart sind. Besonders Besonders bevorzugte Tonmaterialien sind Kaolinit, Illit, Halloysit, Montmorillonit sowie Talk.

Weiterhin sind als anorganische Feinteilchen die Metallsalze der Mono-, Oligo- und Polyphosphorsäuren erfindungsgemäß bevorzugt. Hierunter sind insbesondere die Hydrate bevorzugt, wobei die Mono- bis Deca-Hydrate und Tri-Hydrate besonders bevorzugt sind. Als Metalle kommen insbesondere Alkali- und Erdalkalimetalle in Betracht, wobei die Erdalkalimetalle bevorzugt sind. Hierunter sind Mg und Ca bevorzugt und Mg besonders bevorzugt. Im Zusammenhang mit Phosphaten, Phosphorsäuren und deren Metallverbindungen wird auf Hollemann und Wiberg, Lehrbuch der Anorganischen Chemie, Walter de Gruyter-Verlag, 91.-100. Auflage, 1985, auf den Seiten 651 bis 669 verwiesen.

Bevorzugte kohlenstoffhaltige, jedoch nicht organische Hilfsstoffe sind diejenigen reinen Kohlenstoffe, die in Hollemann und Wiberg, Lehrbuch der Anorganischen Chemie, Walter de Gruyter-Verlag, 91.-100. Auflage, 1985 auf den Seiten 705 bis 708 als Graphite genannt sind. Besonders bevorzugte Graphite sind künstliche Graphite wie beispielsweise Koks, Pyrographit, Aktivkohle oder Ruß.

Die im erfindungsgemäßen Verfahren erhaltenen wasserabsorbierende Polymere sind vorzugsweise dadurch erhältlich, dass zunächst aus den vorgenannten Monomeren und Vernetzern ein Hydrogel-Polymer in partikulärer Form hergestellt wird. Die Herstellung dieses Ausgangsmaterials für die wasserabsorbierenden Polymere erfolgt beispielsweise durch Massepolymerisation, die vorzugsweise in Knetreaktoren wie Extrudern erfolgt, Lösungspolymerisation, Spraypolymerisation, inverse Emulsionspolymerisation oder inverse Suspensionspolymerisation. Bevorzugt wird die Lösungspolymerisation in Wasser als Lösemittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818.

Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Hierzu gehören thermische Initiatoren, Redoxinitiatoren und Photoinitiatoren, deren Aktivierung durch energiereiche Strahlung erfolgt. Die Polymerisationsinitiatoren können dabei in einer Lösung erfindungsgemäßer Monomere gelöst oder dispergiert enthalten sein. Bevorzugt ist der Einsatz wasserlöslicher Initiatoren.

Als thermische Initiatoren kommen sämtliche dem Fachmann bekannte, unter Temperatureinwirkung in Radikale zerfallende Verbindungen in Betracht. Besonders bevorzugt sind dabei thermische Polymerisationsinitiatoren mit einer Halbwertszeit von weniger als 10 Sekunden, darüber hinaus bevorzugt von weniger als 5 Sekunden bei weniger als 180 ºC, darüber hinaus bevorzugt bei weniger als 140ºC. Dabei sind Peroxide, Hydroperoxide, Wasserstoffperoxid, Persulfate sowie Azoverbindungen besonders bevorzugte thermische Polymerisationsinitiatoren. In manchen Fällen ist es vorteilhaft, Mischungen verschiedener thermischer Polymerisationsinitiatoren zu verwenden. Unter diesen Mischungen sind die aus Wasserstoffperoxid und Natrium- oder Kaliumperoxodisulfat bevorzugt, die in jedem denkbaren Mengenverhältnis eingesetzt werden können. Geeignete organische Peroxide sind vorzugsweise Acetylacetonperoxid, Methylethylketonperoxid, Benzoylperoxid, Lauroylperoxid, Acetylperoxid, Capyrlperoxid, Isopropylperoxydicarbonat, 2-Ethylhexylperoxydicarbonat, t-Butylhydroperoxid, Cumolhydroperoxid, t-Amylperpivalat, t-Butylperpivalat, t-Butylperneohexonat, t-Butylisobutyrat, t-Butylper-2-ethylhexenoat, t-Butylperisononanoat, t-Butylpermaleat, t-Butylperbenzoat, t-Butyl-3,5,5-tri-methylhexanoat und Amylperneodekanoat. Weiterhin sind als thermische Polymerisationsinitiatoren bevorzugt: Azo-Verbindungen, wie Azobisisobutyronitrol, Azobisdimethylvaleronitril, 2,2'-Azobis-(2-amidinopropan)dihydrochlorid, Azo-bis-amidinopropan-dihydrochlord, 2,2'-Azobis-(N,N-dimethylen)isobutyramidin-dihydrochlorid, 2- (Carbamoylazo)- isobutyronitril und 4,4'-Azobis-(4-cyanovaleriansäure). Die genannten Verbindungen werden in üblichen Mengen eingesetzt, vorzugsweise in einem Bereich von 0,01 bis 5, bevorzugt von 0,1 bis 2 Mol-%, jeweils bezogen auf die Menge der zu polymerisierenden Monomere.

Die Redoxinitiatoren enthalten als oxidische Komponente mindestens eine der oben angegebenen Perverbindungen und als reduzierende Komponente vorzugsweise Ascorbinsäure, Glukose, Sorbose, Manose, Ammonium- oder Alkalimetall-hydrogensulfit, - sulfat, -thiosulfat, -hyposulfit oder -sulfid, Metallsalze, wie Eisen-II-ionen oder Silberionen oder Natriumhydroxymethylsulfoxylat. Vorzugsweise wird als reduzierende Komponente des Redoxinitiators Ascorbinsäure oder Natriumpyrosulfit verwendet. Bezogen auf die bei der Polymerisation eingesetzte Menge an Monomeren wird 1×10⁻⁵ bis 1 Mol-% der reduzierenden Komponente des Redoxinitiators und 1×10⁻⁵ bis 5 Mol-% der oxidierenden Komponente des Redoxinitiators eingesetzt. Anstelle der oxidierenden Komponente des Redoxinitiators, oder in Ergänzung zu diesem, können ein oder mehrere, vorzugsweise wasserlösliche, Azoverbindungen verwendet werden.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte α-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)malein- imid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclo-hexanon und 2,6-Bis-(p-azidobenzyliden)-4-methylcyclohexanon. Die Photoinitiatoren werden, falls sie eingesetzt werden, üblicherweise in Mengen von 0,01 bis 5 Gew.-%, bezogen auf die zu polymerisierenden Monomeren angewendet.

Bevorzugt wird erfindungsgemäß ein Initiatorsystem bestehend aus Wasserstoffperoxid, Natriumperoxodisulfat und Ascorbinsäure eingesetzt. In der Regel wird die Polymerisation mit den Initiatoren in einem Temperaturbereich von 0°C bis 90°C initiiert.

Die Polymerisationsreaktion kann durch einen Initiator oder durch mehrere, zusammenwirkende Initiatoren ausgelöst werden. Weiterhin kann die Polymerisation derart durchgeführt werden, dass man zunächst ein oder mehrere Redoxinitiatoren zusetzt. Im weiteren Polymerisationsverlauf werden dann zusätzlich thermische Initiatoren oder Photoinitiatoren appliziert, wobei im Falle von Photoninitiatoren die Polymerisationsreaktion dann durch die Einwirkung energiereicher Strahlung initiiert wird. Auch die umgekehrte Reihenfolge, also die anfängliche Initiierung der Reaktion mittels energiereicher Strahlung und Photoinitiatoren oder thermischen Initiatoren und eine im weiteren Polymerisationsverlauf erfolgende Initiierung der Polymerisation mittels eines oder mehrerer Redoxinitiatoren ist denkbar.

Um die so erhaltenen Hydrogel-Polymeren in eine partikuläre Form zu überführen, können diese nach ihrer Abtrennung aus der Reaktionsmischung, bevorzugt nach dem sie zerkleinert und oder gewolft wurden, zunächst bei einer Temperatur in einem Bereich von 20 bis 300ºC, bevorzugt in einem Bereich von 50 bis 250ºC und besonders bevorzugt in einem Bereich von 100 bis 200ºC bis hin zu einem Wassergehalt von weniger als 40 Gew.-%, bevorzugt von weniger als 20 Gew.-% und darüber hinaus bevorzugt von weniger als 10 Gew.-%, jeweils bezogen auf das Gesamtgewicht des Hydrogel-Polymers, getrocknet werden. Die Trocknung erfolgt vorzugsweise in dem Fachmann bekannten Öfen oder Trockner, beispielsweise in Bandtrocknern, Hordentrocknern, Drehrohröfen, Wirbelbetttrocknern, Tellertrocknern, Paddeltrocknern oder Infrarottrocknern.

Gemäß der vorliegenden Erfindung erfolgt das Zerkleinern oder Mahlen dabei vorzugsweise durch Trockenmahlen, vorzugsweise durch Trockenmahlen in einer Hammermühle, einer Stiftmühle, einer Kugelmühle oder einer Walzenmühle. In einer weiteren Ausführung der vorliegenden Erfindung kann die Zerkleinerung des Hydrogel-Polymeren auch durch die Kombinationen von mehreren der vorbeschriebenen Mühlen erfolgen.

In einer bevorzugten Ausführungsform der erfindungsgemäßen Verfahren werden als wasserabsorbierende Polymere, Partikel erhalten, die einen Innenbereich und einen den Innenbereich begrenzenden Oberflächenbereich aufweisen. Wobei der Oberflächenbereich eine andere chemische Zusammensetzung als der Innenbereich aufweist oder sich in einer physikalischen Eigenschaft vom Innenbereich unterscheidet. Physikalische Eigenschaften, in denen sich der Innenbereich vom Oberflächenbereich unterscheidet, sind beispielsweise die Ladungsdichte oder der Vernetzungsgrad.

Diese einen Innenbereich und einen den Innenbereich begrenzenden Oberflächenbereich aufweisenden wasserabsorbierenden Polymere sind vorzugsweise dadurch erhältlich, dass oberflächennahe, reaktive Gruppen der Partikel des partikulären Hydrogel-Polymers (VP) nachvernetzt werden. Diese Nachvernetzung kann thermisch, photochemisch oder chemisch erfolgen.

Als Nachvernetzer bevorzugt sind die im Zusammenhang mit den Vernetzern (α3) genannten Verbindungen der Vernetzerklasse II und IV.

Unter diesen Verbindungen sind als Nachvernetzer besonders bevorzugt Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on.

Besonders bevorzugt wird Ethylencarbonat als Nachvernetzer eingesetzt.

Bevorzugte Ausführungsformen der wasserabsorbierenden Polymere sind diejenigen, die durch Vernetzer der folgenden Vernetzerklassen bzw. durch Vernetzer der folgenden Kombinationen von Vernetzerklassen nachvernetzt sind: II, IV und II IV.
Vorzugsweise wird der Nachvernetzer in einer Menge in einem Bereich von 0,01 bis 30 Gew.-%, besonders bevorzugt in einer Menge in einem Bereich von 0,1 bis 20 Gew.-% und darüber hinaus bevorzugt in einer Menge in einem Bereich von 0,3 bis 5 Gew.-%, jeweils bezogen auf das Gewicht der superabsorbierenden Polymere bei der Nachvernetzung eingesetzt.

Es ist ebenfalls bevorzugt, dass die Nachvernetzung dadurch erfolgt, dass ein Lösemittel, umfassend vorzugsweise Wasser, mit Wasser mischbare organische Lösemittel wie etwa Methanol oder Ethanol oder Mischungen aus mindestens zwei davon, sowie den Nachvernetzer mit dem Aussenbereich der Hydrogel-Polymerteilchen in Kontakt gebracht werden. Die Hydrogel-Polymerteilchen werden bevorzugt anschließend auf eine Temperatur in einem Bereich von 30 bis 300°C, besonders bevorzugt in einem Bereich von 100 bis 200°C gebracht, um die Nachvernetzungsreaktion abzuschließen und um überschüssiges Wasser und oder Lösungsmittel zu entfernen (Trockenen). Das in Kontakt bringen erfolgt dabei vorzugsweise durch Aufsprühen, der Mischung bestehend aus Nachvernetzer und Lösemittel, auf die Hydrogel-Polymerteilchen und anschließendes Mischen der mit der Mischung in Kontakt gebrachten Hydrogel-Polymerteilchen. Dabei ist der Nachvernetzer in der Mischung vorzugsweise in einer Menge in einem Bereich von 0,01 bis 20 Gew.-%, besonders bevorzugt in einer Menge in einem Bereich von 0,1 bis 10 Gew.-%, bezogen auf das Gesamtgewicht der Mischung, enthalten. Es ist weiterhin bevorzugt, dass in einer Menge in einem Bereich von 0,01 bis 50 Gew.-%, besonders bevorzugt in einer Menge in einem Bereich von 0,1 bis 30 Gew.-%, jeweils bezogen auf das Gewicht der Hydrogel-Polymerteilchen, mit den Hydrogel-Polymerteilchen in Kontakt gebracht wird.

Als Kondensationsreaktionen kommen vorzugsweise die Bildung von Ester-, Amid-, Imid- oder Urethanbindungen in Betracht, wobei die Bildung von Esterbindung bevorzugt ist.

Darüber hinaus können dem erfindungsgemäßen Hydrogel-Polymeren und/oder wasserabsorbierenden Polymeren weitere Additive, beispielsweise solche, die als Effektstoffe fungieren, zugesetzt werden. Additive können dem wasserabsorbierenden Polymer in jedem Verfahrensschritt, beispielsweise der Monomerlösung, dem ungetrockneten Gel, dem getrockneten Polymer oder vor oder nach der Nachvernetzung, zugefügt werden. Es kann vorteilhaft sein, Additive, die wärme- oder abrasionsempfindlich oder empfindlich gegenüber bestimmten Verfahrensschritten sind, nach dem Nachvernetzungsschritt als einer der letzten Prozessschritte zuzuführen. Verfahrensschritte, die nach dem Nachvernetzungsschritt durchgeführt werden, werden allgemein auch Konfektionierung bezeichnet.

Als Additive sind des weiteren Trennmittel bevorzugt, wie etwa anorganische oder organische pulverförmige Trennmittel. Diese Trennmittel werden vorzugsweise in Mengen in einem Bereich von 0 bis 2 Gew.-%, besonders bevorzugt in einem Bereich von 0,1 bis 1,5 Gew.-%, bezogen auf das Gewicht des Hydrogel-Polymers und/oder des wasserabsorbierenden Polymers, eingesetzt. Bevorzugte Trennmittel sind Holzmehl, Pulp Fasern, pulverförmige Rinde, Cellulosepulver, mineralische Füllstoffe wie Perlit, synthetische Füllstoffe wie Nylonpulver, Rayonpulver, Diatomerde, Bentonit, Kaolin, Zeolithe, Talk, Lehm, Asche, Kohlenstaub, Magnesiumsilikate, Dünger oder Mischungen der Substanzen. Hochdisperse pyrogene Kieselsäure wie sie unter dem Handelsnamen Aerosil von Evonik Degussa vertrieben wird ist bevorzugt.

In einer weiteren bevorzugten Ausführungsform des erfindungsgemäßen Verfahrens erfolgt das in Kontakt bringen der Hydrogel-Polymerteilchen und oder der wasserabsorbierenden Polymerteilchen mit einen Effektstoff, wie z.B. einem Polyzucker, einer polyphenolischen Verbindung wie z.B. hydrolisierbare Tannine oder einer Silizium-Sauerstoff beinhaltenden Verbindung, keimhemmenden Mitteln, Enzyminhibitoren, Geruchshemmer, Geruchsinhibitoren, Antitranspirantien oder einer Mischung von mindestens zwei darauf basierenden Effektstoffen. Der Effektstoff kann sowohl in fester Form (Pulver) als auch in gelöster Form mit einem Lösemittel zugegeben werden, wobei die Zugabe des Effektstoffs frühestens nach dem Verfahrensschritt iii) erfolgt. Als Effektstoff wird im Rahmen der vorliegenden Erfindung ein Stoff verstanden, der zur Geruchsinhibierung dient.

Erfindungsgemäße Polyzucker sind solche aus der Gruppe von den geläufigen Stärken und deren Derivate, Cellulosen und deren Derivate, Cyclodextrine. Wobei als Cyclodextrine vorzugsweise α-Cyclodextrin, β-Cyclodextrin, γ-Cyclodextrin oder Mischungen aus diesen Cyclodextrinen verstanden werden.

Als Silizium-Sauerstoff beinhaltende Verbindungen sind Zeolithe bevorzugt. Als Zeolithe können alle dem Fachmann bekannten synthetischen oder natürlichen Zeolithe eingesetzt werden. Bevorzugte natürliche Zeolithe sind Zeolithe aus der Natrolith-Gruppe Harmoton-Gruppe, der Mordenit-Gruppe, der Chabasit-Gruppe, der Faujasit-Gruppe (Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabazit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmotom, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS®.

Als Kationen enthalten die in dem erfindungsgemäßen Verfahren eingesetzten Zeolithe vorzugsweise Alkalimetall-Kationen wie Li⁺, Na⁺, K⁺, Rb⁺, Cs⁺ oder Fr⁺ und/oder Erdalkalimetall-Kationen wie Mg²⁺, Ca²⁺, Sr²⁺ oder Ba²+.

Als Zeolithe können Zeolithe des sogenannten "mittleren" Typs eingesetzt werden, bei denen das SiO₂/AlO₂-Verhältnis kleiner als 10 ist, besonders bevorzugt liegt das SiO₂/AlO₂-Verhältnis dieser Zeolithe in einem Bereich von 2 bis 10. Neben diesen "mittleren" Zeolithen können weiterhin Zeolithe des "hohen" Typs eingesetzt werden, zu denen beispielsweise die bekannten "Molekularsieb"-Zeolithe des Typs ZSM sowie beta-Zeolith gehören. Diese "hohen" Zeolithe sind vorzugsweise durch ein SiO₂/AlO₂-Verhältnis von mindestens 35, besonders bevorzugt von einem SiO₂/AlO₂-Verhältnis in einem Bereich von 200 bis 500 gekennzeichnet.

Vorzugsweise werden die Zeolithe als Partikel mit einer mittleren Partikelgröße in einem Bereich von 1 bis 500 µm, besonders bevorzugt in einem Bereich von 2 bis 200 µm und darüber hinaus bevorzugt in einem Bereich von 5 bis 100 µm eingesetzt.

Die Effektstoffe werden in den erfindungsgemäßen Verfahren vorzugsweise in einer Menge in einem Bereich von 0,1 bis 50 Gew.-%, besonders bevorzugt in einem Bereich von 1 bis 40 Gew.-% und darüber hinaus bevorzugt in einer Menge in einem Bereich von 5 bis 30 Gew.-%, jeweils bezogen auf das Gewicht der Hydrogel-Polymerteilchen und oder wasserabsorbierenden Polymerteilchen, eingesetzt.

Als keimhemmende Mittel sind grundsätzlich alle gegen grampositive Bakterien wirksamen Stoffe bevorzugt, wie z. B. 4-Hydroxybenzoesäure und ihre Salze und Ester, N-(4-Chlorphenyl)-N'-(3,4dichlorphenyl)harnstoff,2,4,4'-Trichlor-2'-hydroxydiphenylether (Triclosan), 4-Chlor-3,5-dimethylphenol, 2,2'- Methylen-bis(6-brom-4-chlorphenol), 3-Methyl-4-(1-methylethyl)phenol, 2-Benzyl-4-chlorphenol, 3-(4- Chlorphenoxy)-1,2-propandiol, 3-lod-2-propinylbutylcarbamat, Chlorhexidin, 3,4,4'-Trichlorcarbonilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen TM CAT, Cognis GmbH, Düsseldorf/Deutschland). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben im Allgemeinen keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, alpha -Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, beta-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien reduzieren durch Beeinflussung der Aktivität der ekkrinen Schweißdrüsen die Schweißbildung, und wirken somit Achselnässe und Körpergeruch entgegen. Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium- Zirkonium-Trichlorohydrat, Aluminium-Zirkonium-Tetrachlorohydrat, Aluminium-Zirkonium-Pentachlorohydrat und deren Komplexverbindungen z. B. mit Aminosäuren wie Glycin.

Als weitere Additive im Rahmen der vorliegenden Erfindung seien beispielhaft noch Anti-Caking-Verbindungen wie z.B. Kaolin, Aerosile®, Sipernate®, und ähnliche, unlösliche anorganischen Additive auf Siliciumbasis wie z.B. Kieselsäuren oder Kieselsäuresole, Aluminiumsalze wie Aluminiumsulfat oder Aluminiumlactat, Surfactants, wie z.B. Tenside, Viskisitätsmodifizierungsmittel oder ähnliche erwähnt die auf der Oberfläche der Polymerpartikel aufgebracht werden oder aber auch mit den freien Polymerketten des Polymerpartikels reagieren.

Zum Mischen bzw. Besprühen sind alle Vorrichtungen geeignet, die eine homogene Verteilung einer Lösung, Pulver, Suspension oder Dispersion auf oder mit den Hydrogel-Polymerteilchen oder wasserabsorbierenden Polymeren erlauben. Beispiele sind Lödige Mischer (hergestellt durch die Firma *Gebrüder Lödige Maschinenbau GmbH*), Gericke Multi-Flux Mischer (hergestellt durch die Firma *Gericke GmbH*)*,* DRAIS-Mischer (hergestellt durch die Firma *DRAIS GmbH* Spezialmaschinenfabrik Mannheim), Hosokawa Mischer (*Hosokawa Mokron Co., Ltd.*)*,* Ruberg Mischer (hergestellt durch die Firma *Gebr. Ruberg GmbH & C0.KG Nieheim),* Hüttlin Coater (hergestellt durch die Firma *BWI Hüttlin GmbH Steinen*), Fließbetttrockner oder Sprühgranulatoren von AMMAG (hergestellt durch die Firma *AMMAG Gunskirchen, Österreich*) oder Heinen (hergestellt durch die Firma *A. Heinen AG Anlagenbau Varel*), Patterson-Kelly-Mischer, NARA-Schaufelmischer, Schneckenmischer, Tellermischer, Wirbelschichttrockner oder Schugi-Mischer. Für das in Kontakt bringen in einer Wirbelschicht können alle dem Fachmann bekannten und geeignet erscheinenden Wirbelschichtverfahren angewandt werden. Beispielsweise kann ein Wirbelschichtcoater eingesetzt werden.

In einer weiteren bevorzugten Ausführungsform wird die Peroxoverbindung in einer Menge von 0,0001 bis 1,5 Gew.-%, bezogen auf die Acrylsäure nach der Polymerisation eingesetzt.

In einer weiteren bevorzugten Ausführungsform wird erfindungsgemäß die Peroxoverbindung, in den Schritten (iv) bis (vii) zugesetzt.

In einer weiteren besonders bevorzugten Ausführungsform wird erfindungsgemäß die Peroxoverbindung, im Schritt (vii) zugesetzt.

Offenbart wird hier auch ein Verbund, beinhaltend die durch die erfindungsgemäßen Verfahren erhältlichen wasserabsorbierenden Polymere oder Hydrogel-Polymere und ein Substrat. Es ist dabei bevorzugt, dass die wasserabsorbierenden Polymere oder Hydrogel-Polymere und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder Schäume bevorzugt. Weiterhin ist es bevorzugt, dass der Verbund mindestens einen Bereich umfasst, welcher wasserabsorbierenden Polymere oder Hydrogel-Polymere in einer Menge im Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm³, vorzugsweise mindestens 0,1 cm³ und am meisten bevorzugt mindestens 0,5 cm³ aufweist.

Offenbart wird auch ein Verfahren zur Herstellung eines Verbundes, wobei die die durch das erfindungsgemäße Verfahren erhältlichen Superabsorber und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem Verbund genannt wurden.

Offenbart werden auch chemische Produkte beinhaltend die wasserabsorbierenden Polymere oder Hydrogel-Polymere oder einen Verbund. Bevorzugte chemische Produkte sind insbesondere Schäume, Formkörper, Fasern, Folien, Filme, Kabel, Dichtungsmaterialien, flüssigkeitsaufnehmende Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der wasserabsorbierenden Polymere oder des Verbundes in chemischen Produkten, vorzugsweise in den vorstehend genannten chemischen Produkten, insbesondere in Hygieneartikeln wie Windeln, oder Damenbinden, sowie die Verwendung der wasserabsorbierenden Polymerpartikel als Träger für pflanzen- oder pilzwachstumsregulierende Mittel oder Pflanzenschutzwirkstoffe werden hier offenbart. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

### TESTMETHODEN:

Sofern nicht nachfolgend anders angegeben, werden die hierin erfolgten Messungen nach ERT-Verfahren. "ERT" steht für *EDANA Recommended Test* und "EDANA" für *European Disposable and Nonwoven Association.* Alle Testmethoden werden grundsätzlich, wenn nichts anderes angegeben ist, bei einer Umgebungstemperatur von 23±2 °C und einer relativen Luftfeuchte von 50±10 % durchgeführt werden.

### Partikelgrößeverteilung (PSD Particle Size Distribution):

Die Partikelgrößenverteilung der wasserabsorbierenden Polymerpartikel wird analog der von der EDANA empfohlenen Testmethode Nr. WSP 220.3-10 "Particle Size Distribution" bestimmt.

### Zentrifugenretentionskapazität (CRC Centrifuge Retention Capacity):

Die Bestimmung der Zentrifugenretentionskapazität erfolgte gemäß der EDANA (European Disposables and Nonwovens Association) empfohlenen Testmethode Nr. WSP 241.3-10 "Centrifuge retention capacity".

### Absorption gegen einen Druck von 0,7 psi (AAP):

Die Absorption unter Druck wurde als AAP (Absorption Against Pressure) nach WSP 242.3-10 an der gesamten Kornfraktion bestimmt. Entsprechendwurden0,90g der Prüfsubstanz (abgesiebt zwischen 150 und 850µm) in einen Testzylinder mit 60,0 mm Innendurchmesser und einem Siebboden (400mesh) eingewogen (Konzentration: 0,032 g/cm²) und gleichmäßig verteilt. Auf die Prüfsubstanz wird ein zylindrisches Gewicht 50 g/cm² = 0,7psi) mit einem Außendurchmesser von 59,2 mm gelegt. In eine Kunststoffschale werden Filterplatten gelegt, die mit einem Filterpapier abgedeckt werden. Die Kunststoffschale wird mit 0,9 %iger NaCl-Lösung gefüllt, bis der Flüssigkeitsspiegel mit der Oberkante der Filterplatten abschließt. Anschließend werden die vorbereiteten Messeinheiten auf die Filterplatten gestellt. Nach einer Quellzeit von 60 Minuten werden die Messeinheiten entnommen und das Gewicht entfernt. Die aufgenommene Flüssigkeitsmenge wird gravimetrisch ermittelt und auf 1 Gramm Prüfsubstanz umgerechnet.

### Bestimmung der Ammoniak (NH3)-Freisetzung (Proteus mirabilis):

Proteus mirabilis wurde über Nacht bei 37°C auf einem Caso-Schrägagar angezogen. Die Bakterienkultur wurde mit 5 ml synthetischem Urin (25 g/l Harnstoff, 9 g/l Natriumchlorid, 5 g/l Glukose, 4 g/l Kaliumsulfat, 2,5 g/l Ammoniumsulfat, 0,7 g/l Calciumacetat, 0,7 g/l Magnesiumsulfat x 7 H₂O, 0,5 g/l Hefe-Extrakt, 5g/l Fleischextrakt, 5g/l Pepton) abgeschwemmt. Die Keimzahl des synthetischen Urins wurde so eingestellt, dass eine Anfangskeimzahl von ca. 10⁵ KbE/ml Urin vorhanden war. Jeweils 33 ml des mit Bakterien versetzten künstlichen Urins wurden in Erlenmeyerkolben überführt und mit 1 g Superabsorber versetzt. Die Gefäße wurden mit einem Gummistopfen, durch dessen Bohrung ein Dräger - Diffusionsröhrchen (Ammoniak 20/a-D) geführt wurde, verschlossen und bei 37°C in einen Brutschrank inkubiert. Der freigesetzte Ammoniak wurde in ppm x h gemessen.

### Bestimmung der Geruchsreduzierung von Organischen Geruchsstoffen:

### Mittels Solid Phase Microextration (SPME)- Gas Chromatography (GC)

Mit diesem Test wurde die Geruchsreduzierung von sechs erschiedenen organischen Substanzen getestet, die unterschiedliche chemische Substanzklassen repräsentieren.

### SPME-GC

0,50 g des zu bestimmenden Superabsorber werden in einen 200 ml Erlenmeyerkolben genau eingewogen und mit 11,0g eines Ansatzes bestehend aus 100 ng/ml, Dimethyltrisulfid, 0,5 ng/ml, Pyrrol, 50 ng/ml Furfurylmercaptan, 0,5 ng/ml (S)-(+) Carvon, 0,5 ng/ml Indol und 13000 ng/ml Trimethylamin in einer 0,9 gew.% wässrigen NaCl-Lösung beaufschlagt. Der Kolben wird mit einem Gewindeadapter mit Septum verschlossen und 16h zur Gleichgewichtseinstellung des Dampfraums bei 37°C im Klimaschrank gelagert. Jetzt wird die SPME-Phase für 30 min. in den Dampfraum gebracht und anschließend direkt in einen Gaschromatograph zur Desorption und Analyse des Anteils an Schlechtgerüchen injiziert. Bestimmt wird die Abnahme bzw. die Reduktion an Konzentration des Geruchsstoffes ins Verhältnis gesetzt zu dem entsprechenden Referenzmuster in %.

**Geräteparameter:**

| | | |
|---|---|---|
| Kolben: | 200ml Erlenmeyerkolben mit NS 29 = Volumen 255ml mit Gewindeadapter und Septum | |
| SPME Phase: | Supelco Carboxen / PDMS-schwarz | |
| GC-Säule: | RESTEK Corp. RTX-50, 30m, 0,53 | |
| Trägergas: | He | |
| GC: | Hewlett Packard 5890 | |
| Heizraten: | 7 min. | 30°C |
| | 10°C / min. auf | 180°C |
| | 30°C / min. auf | 250°C |

### Beispiele:

Die nachfolgenden Beispiele dienen der näheren Erläuterung der Erfindung ohne sie jedoch hierauf zu beschränken.

Für die nachfolgenden erfindungsgemäßen Beispiele wurde eine definierte Partikelgrößenverteilung (PSD) verwendet (150µm bis 850µm).
Unter dem Begriff "SX", wie er in der Beschreibung verwendet wird, versteht man die thermische Oberflächennachvernetzung des Vorproduktes (VP). Das Vorprodukt entspricht dem erzeugten Hydrogel-Polymer nach der ersten Trocknung und Mahlen, mit der vorgenannten Kornverteilung.

### Beschreibung der Herstellung der SAP Proben:

### Polymergebilde (Pulver A)

Eine Monomerlösung bestehend aus 300 g Acrylsäure, neutralisiert zu 60 Mol-% mit Natronlauge mittels 200,2 g 50%ige NaOH), 474,8 g Wasser, 1,62 g Polyethylenglykol-300-diacrylat, 0,89 g Monoallylpolyethylenglykol-450-monoacrylsäureester wurde durch Entgasung mit Stickstoff vom gelösten Sauerstoff befreit und auf die Starttemperatur von ca. 4°C abgekühlt. Nachdem dem Erreichen der Starttemperatur, erfolgt die Zugabe der Initiatorlösung bestehend aus 0,3 g Natriumperoxodisulfat in 10 g Wasser, 0,07 g 35%ige Wasserstoffperoxidlösung in 10 g Wasser und 0,015 g Ascorbinsäure in 2 g Wasser. Eine exotherme Polymerisationsreaktion fand statt. Die adiabatische Endtemperatur betrug ca. 100 °C. Das entstandene Hydrogel wurde mit einem Labor-Fleischwolf zerkleinert (5mm Lochscheibe)und die zerkleinerte Probe 90 Minuten bei 170°C im Labor-Umlufttrockenschrank getrocknet. Das getrocknete Polymerisat wurde zunächst grob zerstoßen und anschließend mittels einer Schneidmühle SM100 mit einer 2mm Korndurchlochung gemahlen und auf ein Pulver mit einer Partikelgröße von 150 bis 850 µm gesiebt. 100 g des Pulvers wurden mit einer Lösung aus 1,0 g Ethylencarbonat und 3,0 g entionisiertes Wasser beschichtet. Dabei wurde die Lösung mittels einer Spritze (0.45 mm Kanüle) auf das sich im Mischer befindliche Polymersatpulver aufgetragen. Das beschichtete Pulver erhitze man anschließend im Trockenschrank bei 170 °C über einen Zeitraum von 90 Minuten.

### Beispiel 1, Referenzprobe:

Als Referenzprobe dient Pulver A ohne weitere zusätzliche Behandlung.

### Beispiel 2:

100 g vom Pulver A wurden mit 0,5 g Kaliumperoxomonosulfat - Triple Salz (Caro'sche Säure) versetzt und ca. 2 Stunden auf dem Überkopfschüttler homogenisiert.

### Beispiel 3:

100 g vom Pulver A wurden mit 1,0 g Kaliumperoxomonosulfat - Triple Salz versetzt und ca. 2 Stunden auf dem Überkopfschüttler homogenisiert.

### Ammoniak - Geruchskontrolle:

| Probenbezeichnung | Retention [g/g] | AAP 0.7 psi [g/g] | NH3 - Geruchskontrolle [h] |
|---|---|---|---|
| Referenzprobe, Beispiel 1 | 30.2 | 21.2 | 6-7 |
| Beispiel 2 | 31.0 | 20.4 | 18-20 |
| Beispiel 3 | 31.2 | 20.3 | 20-22 |

Wie die Ergebnisse zeigen eignen sich die erfindungsgemäßen, geruchskontrollierenden, superabsorbierenden Polymerpartikel hervorragend, um die Entstehung von Ammoniak-Verbindungen bis zu 22 Stunden zu verhindern, bei gleichzeitiger Aufrechterhaltung exellenter Absorptionseigenschaften des Polymers. Der Vergleich hingegen zeigt, dass sich bereits nach 6 bis 7 Stunden Ammoniak bildet.

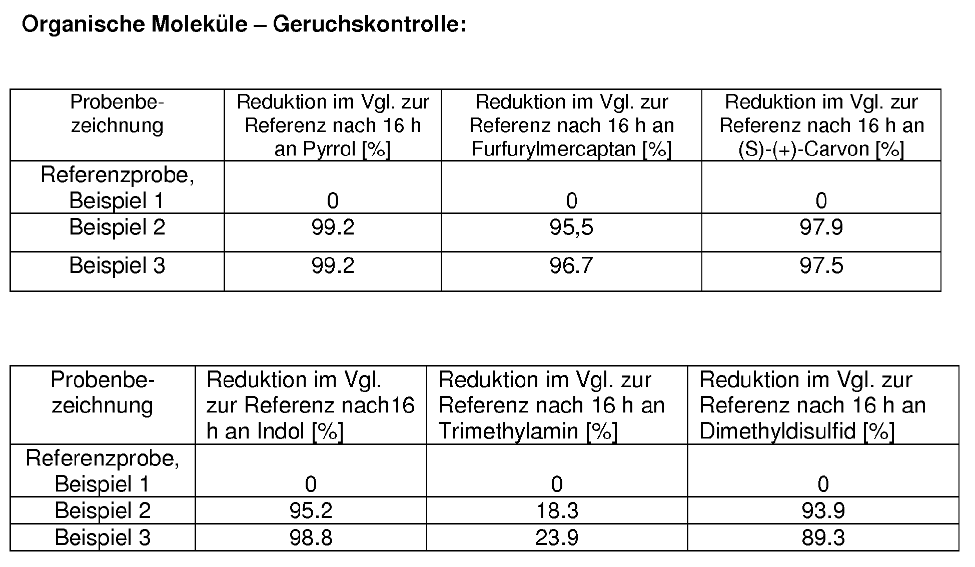

Wie die Beispiele klar zeigen, ermöglichen die erfindungsgemäßen, geruchskontrollierenden, superabsorbierenden Polymerpartikel nicht nur die Kontrolle über Schlechtgerüche aus Ammoniak-Verbindungen, sondern ebenfalls exzellente Kontrolle von Schlechtgerüchen aus organischen Verbindungen. Als unangenehm riechende organische Substanzen wurden Pyrrol, Furfurylmercaptan, (S)-(+)-Carvon, Indol, Trimethylamin und Dimethyldisulfild eingesetzt, die alle sechs allgemein bekannte Schlechtgerüche darstellen und zudem sechs unterschiedliche chmische Substanzklassen repräsentieren. Die erfindungsgemäßen, geruchskontrollierenden, superabsorbierenden Polymerpartikel sind in der Lage, Schlechtgerüche bis zu 99% zu eliminieren.

Beispiele 2 und 3 wurden mit äquivalenten Mengen verschiedener Salze von Peroxomonosulphat, Peroxodisulphat und Sulphomonopersäure wiederholt. Alle getesteten Peroxoverbindungen wiesen exzelente Eigenschaften bei der Kontrolle und Inhibierung von Schlechtgerüchen auf und erzielten ähnliche Ergebnisse wie den Tabellen vorab angezeigten.

Darüber hinaus wurde die Peroxoverbindung Kaliumperoxomonosulfat - Triple-Salz mit einer Vielzahl unterschiedlicher Superabsorber Muster analog zu den vorab beschriebenen Versuchen mit Pulver A getestet. Alle getesteten erfindungsgemäßen, geruchskontrollierenden, superabsorbierenden Polymerpartikel basierend auf unterschiedlichen Monomerkonzentationen, unterscheidlichen Vernetzern, untersccheidlichen Nachvernetzern und unerschiedlichen Herstellungsbedingungen, welche wie in Beispiel 2 oder 3 beschieben behandelt wurden, wiesen hervorragende Ergebnisse in der Inhibierung von Ammoniak-Gerüche bis zu 22 Stunden und bei der Inhibierung von Schlechtgerüchen aus organischen Verbindungen.

## Patentansprüche

1. Verfahren zur Herstellung wasserabsorbierender Polymere, umfassend die Verfahrenschritte
**(i)** Mischen von
(α1) 0,1 bis 99,999 Gew.-%, bevorzugt 20 bis 98,99 Gew.-% und besonders bevorzugt 30 bis 98,95 Gew.-% polymerisierbaren, ethylenisch ungesättigten, säuregruppenhaltigen Monomeren, nämlich Acrylsäure oder deren Salze oder deren Mischungen,
(α2) 0 bis 70 Gew.-%, bevorzugt 1 bis 60 Gew.-% und besonders bevorzugt 1 bis 40 Gew.-% polymerisierbaren, ethylenisch ungesättigten, mit (α1) copolymerisierbaren Monomeren,
(α3) 0,001 bis 10 Gew.-%, bevorzugt 0,01 bis 7 Gew.-% und besonders bevorzugt 0,05 bis 5 Gew.-% eines oder mehrerer Vernetzer,
(α4) 0 bis 30 Gew.-%, bevorzugt 1 bis 20 Gew.-% und besonders bevorzugt 5 bis 10 Gew.-% wasserlöslichen Polymeren,
(α5) 0 - 90 Gew.-%, vorzugsweise 2,5-75 Gew.-% und besonders bevorzugt 10 - 60 Gew.-% Wasser, sowie
(α6) 0-20 Gew.-%, vorzugsweise 0-10 Gew.-% und besonders bevorzugt 0,1-8 Gew.-% eines oder mehrerer Hilfsmittel, wobei die Summe der Gewichtsmengen (α1) bis (α6) 100 Gew.-% beträgt
**(ii)** radikalische Polymerisation unter Vernetzung, um ein wasserunlösliches, wässriges unbehandeltes Hydrogel-Polymer zu bilden,
**(iii)** Zerkleinern des Hydrogel-Polymers
**(iv)** Trocknen des Hydrogel-Polymers,
**(v)** Mahlen und Absieben des Hydrogel -Polymers,
**(vi)** Oberflächennachvernetzung des gemahlenen und gesiebten Hydrogel-Polymers und
**(vii)** Konfektionierung des wasserabsorbierenden Polymers,
wobei
das wasserabsorbierende Polymer mit 0,0001 bis 3 Gew.-%, bevorzugt 0,002 bis 2 Gew.-% und besonders bevorzugt 0,007 bis 1,8 Gew.-% einer Peroxoverbindung, bezogen auf die Acrylsäure nach der Polymerisation behandelt ist" wobei die Peroxoverbindung in den Schritten (iii) bis (vii) zugesetzt wird und wobei es sich bei der Peroxoverbindung um Kaliumperoxomonosulfat - Triple Salz handelt.

2. Verfahren nach Anspruch 1, **dadurch gekennzeichnet, dass** das wasserabsorbierende Polymer ganz besonders bevorzugt mit 0,0001 bis 1,5 Gew.-%, der Peroxoverbindung Kaliumperoxomonosulfat - Triple Salz, bezogen auf die Acrylsäure nach der Polymerisation behandelt ist.

3. Verfahren nach einem der vorhergehenden Ansprüche, **dadurch gekennzeichnet, dass** die Peroxoverbindung besonders bevorzugt im Schritt (vii) zugesetzt wird.

## Claims

1. Process for preparing water-absorbing polymers, comprising the process steps of
(**i**) mixing
(α1) 0.1 to 99.999% by weight, preferably 20 to 98.99% by weight and more preferably 30 to 98.95% by weight of polymerizable, ethylenically unsaturated monomers containing acid groups, namely acrylic acid or salts thereof or mixtures thereof,
(α2) 0 to 70% by weight, preferably 1 to 60% by weight and more preferably 1 to 40% by weight of polymerizable, ethylenically unsaturated monomers copolymerizable with (α1),
(α3) 0.001 to 10% by weight, preferably 0.01 to 7% by weight and more preferably 0.05 to 5% by weight of one or more crosslinkers,
(α4) 0 to 30% by weight, preferably 1 to 20% by weight and more preferably 5 to 10% by weight of water-soluble polymers,
(α5) 0-90% by weight, preferably 2.5-75% by weight and more preferably 10-60% by weight of water, and
(α6) 0-20% by weight, preferably 0-10% by weight and more preferably 0.1-8% by weight of one or more assistants, where the sum of the weights of (α1) to (α6) is 100% by weight,
**(ii)** free-radical polymerization with crosslinking to form a water-insoluble,
aqueous untreated hydrogel polymer,
**(iii)** comminuting the hydrogel polymer,
**(iv)** drying the hydrogel polymer,
**(v)** grinding and sieving the hydrogel polymer to size,
**(vi)** surface postcrosslinking the ground and sieved hydrogel polymer
and
**(vii)** finishing the water-absorbing polymer,
wherein
the water-absorbing polymer has been treated with 0.0001 to 3% by weight, preferably 0.002 to 2% by weight and more preferably 0.007 to 1.8% by weight of a peroxo compound, based on the acrylic acid, after the polymerization, wherein the peroxo compound is added in steps (iii) to (vii) and wherein the peroxo compound is potassium peroxomonosulphate triple salt.

2. Process according to Claim 1, **characterized in that** the water-absorbing polymer has most preferably been treated with 0.0001 to 1.5% by weight of the peroxo compound potassium peroxomonosulphate triple salt, based on the acrylic acid, after the polymerization.

3. Process according to either of the preceding claims, **characterized in that** the peroxo compound is more preferably added in step (vii).

## Revendications

1. Procédé de fabrication de polymères absorbant l'eau, comprenant les étapes de procédé suivantes :
(i) le mélange de
(α1) 0,1 à 99,999 % en poids, de préférence 20 à 98,99 % en poids et de manière particulièrement préférée 30 à 98,95 % en poids de monomères éthyléniquement insaturés, contenant des groupes acides, polymérisables, à savoir l'acide acrylique ou ses sels ou leurs mélanges,
(α2) 0 à 70 % en poids, de préférence 1 à 60 % en poids et de manière particulièrement préférée 1 à 40 % en poids de monomères éthyléniquement insaturés polymérisables, copolymérisables avec (α1),
(α3) 0,001 à 10 % en poids, de préférence 0,01 à 7 % en poids et de manière particulièrement préférée 0,05 à 5 % en poids d'un ou de plusieurs agents de réticulation, (α4) 0 à 30 % en poids, de préférence 1 à 20 % en poids et de manière particulièrement préférée 5 à 10 % en poids de polymères solubles dans l'eau,
(α5) 0 à 90 % en poids, de préférence 2,5 à 75 % en poids et de manière particulièrement préférée 10 à 60 % en poids d'eau, et
(α6) 0 à 20 % en poids, de préférence 0 à 10 % en poids et de manière particulièrement préférée 0,1 à 8 % en poids d'un ou de plusieurs adjuvants, la somme des quantités en poids d' (α1) à (α6) étant de 100 % en poids,
(ii) la polymérisation radicalaire avec réticulation afin de former un polymère hydrogel non traité aqueux insoluble dans l'eau,
(iii) le broyage du polymère hydrogel,
(iv) le séchage du polymère hydrogel,
(v) le broyage et le tamisage du polymère hydrogel,
(vi) la post-réticulation de surface du polymère hydrogel broyé et tamisé, et
(vii) le conditionnement du polymère absorbant l'eau, le polymère absorbant l'eau étant traité après la polymérisation avec 0,0001 à 3 % en poids, de préférence 0,002 à 2 % en poids et de manière particulièrement préférée 0,007 à 1,8 % en poids d'un composé peroxo, par rapport à l'acide acrylique, le composé peroxo étant ajouté aux étapes (iii) à (vii) et le composé peroxo consistant en le sel triple de peroxomonosulfate de potassium.

2. Procédé selon la revendication 1, **caractérisé en ce que** le polymère absorbant l'eau est de manière tout particulièrement préférée traité après la polymérisation avec 0,0001 à 1,5 % en poids du composé peroxo sel triple de peroxomonosulfate de potassium, par rapport à l'acide acrylique.

3. Procédé selon l'une quelconque des revendications précédentes, **caractérisé en ce que** le composé peroxo est de manière particulièrement préférée ajouté à l'étape (vii).
